# EUROPEAN PATENT SPECIFICATION

(11) **EP 2 800 021 B1**
(45) Date of publication and mention of the grant of the patent: **04.09.2019**
(21) Application number: 13305574.9
(22) Date of filing: 30.04.2013
(51) Int. Cl.: G16H 40/63, G16H 30/20

(54) **Method of imaging an implant placed into a human body, adapted implant, and adapted imaging system**
Verfahren zur Abbildung eines in einem menschlichen Körper eingesetzten Implantats, angepasstes Implantat und angepasstes Abbildungssystem
Procédé d'imagerie d'un implant placé dans un corps humain, implant adapté et système d'imagerie adapté

(43) Date of publication of application: 05.11.2014
(73) Proprietor: GENERAL ELECTRIC COMPANY, Schenectady, NY 12345 (US)
(72) Inventor: Muller, Serge, 78533 BUC CEDEX (FR); Puong, Sylvie, 78533 BUC CEDEX (FR)
(74) Representative: Hirsch & Associés

(56) References cited:
- US-A- 5 300 120
- US-A1- 2008 242 944
- US-A1- 2009 012 372
- US-A1- 2013 090 946
- US-B2- 7 333 013
- US-B2- 7 704 282

## Description

### FIELD OF THE INVENTION

The invention relates to a method of imaging an implant placed into a human body, to an implant adapted to such an imaging method, and to an imaging system adapted to such an imaging method.

### BACKGROUND OF THE INVENTION

According to a first prior art, the radiologist or imaging specialist asks the patient to know which kind of implant is placed into his or her human body or to know at least some characteristics of the implant which is placed into his or her human body. However, the patient often does not know these characteristics or does not know them with sufficient detail. With this very incomplete set of information on the implant, the first image(s) of the implant acquired by the radiologist or imaging specialist can be of insufficient quality. So, additional images can be needed before a satisfactory image quality of the imaged implant is obtained.

According to a second prior art, for example disclosed in patent US 841340, it is known a communication system between an implant placed into a human body and a device external to human body. However, there is consideration neither about imaging the implant nor about the related difficulties. Besides, the electronic component included in the implant is active and complex. The implant described there is a pacemaker.

According to a third prior art, for example disclosed in patent US 8403907, it is known a communication system between an implant placed into a human body and a device external to human body. However, there is consideration neither about imaging the implant nor about the related difficulties. Besides, the electronic component included in the implant is active and complex. The implant described there is a drug distributing reservoir.

According to a fourth prior art, for example disclosed in patent US 8248232, it is known a communication system between an implant placed into a human body and a device external to human body. However, there is consideration neither about imaging the implant nor about the related difficulties. Besides, the electronic component included in the implant is active and complex: it is a multipart system including sensor, chip and antenna. The implant described there is a pacemaker or an implantable cardioverter defibrillator.

According to a fifth prior art, for example disclosed in patent US 8083741, it is known a communication system between an implant placed into a human body and a device external to human body. However, there is consideration neither about imaging the implant nor about the related difficulties. Besides, the electronic component included in the implant is active and complex: it includes sensor and can even perform some healing of the broken or fractured bone by electric current emission. The implant described there is an orthopedic implant such as a bone plate.
According to a sixth prior art, disclosed in patent application US2013090946 A1, a process is provided for adapting an imaging parameter depending on a stored information provided by a patient. This method comprises the steps of reading information related to a patient from a memory carried by the patient, adapting, according to said read information, at least one imaging parameter of an imaging device, and imaging the patient. The process does not involve any implant and the relevant information is stored on an support external to the patient, such as an RFID-tagged card.

None of former prior art documents deals with imaging an implant, with the related difficulties, by using a simple way to record information within the implant itself.

### SUMMARY OF THE INVENTION

The object of the present invention is to alleviate at least partly the above mentioned drawbacks.

More particularly, the invention aims to provide for a simple and efficient way to allow for implant imaging being performed correctly, preferably right from the beginning, without requiring specific effort from the patient wearing the implant, such as remembering kind of implant or characteristics of his or her implant or history of his or her implant, and preferably without supplementary intrusion or with a limited additional intrusion into the human body of the patient, implant itself being already such an intrusion.

Therefore, information related to the implant, at least representative of or from which can be derived radiopacity level in case of X-ray imaging or more generally speaking response to imaging signal, is most useful for the imaging system as well as for the imaging specialist, to be known as soon as possible, and to be known preferably before implant imaging starts. Response to imaging signal, depending on the nature of imaging signal, for example X-ray, ultrasound, MRI, can be respectively the transparency, the reflectance, radio frequency signal.

The way proposed by invention to provide such useful information is to record it in the implant itself in a such a way that it is easily readable by a reader external to the human body which human body contains the implant, so that it becomes known to the imaging system and or to the imaging specialist quickly and preferably even before starting imaging the implant.

That way, implant imaging can be improved, preferably even optimized right from the beginning, so that image of the implant is of good quality with a limited imaging signal energy (emitted by the energy source of imaging device to perform imaging), or radiation dose in case of X-ray imaging, received by the human body containing the implant.

This object is achieved with a method of imaging an implant placed into a human body, comprising: reading, with a reader external to said body, information related to said implant and recorded in a memory embedded in said implant, adapting, according to said read information, at least one imaging parameter of an imaging device external to said body, imaging, by said imaging device with said adapted imaging parameter(s), said implant.

Said memory is preferably a memory which is included in a chip, which can be read by a reader external to the implant, and which can advantageously be written on by a device external to the implant so as to add information to be recorded or to update already recorded information. Said memory is advantageously writable, and more advantageously wirelessly writable, by a device external to said implant and external to said human body. In a very simple but not preferred embodiment, said memory could also be information simply printed, such as a code bar, for example on the surface or close to the surface of the implant, and readable through human body by a wavelength to which human body is mostly transparent. In that embodiment, no information update can be achieved without operation or intrusion into said human body. An alternative with this printed information is that it is permanent information which corresponds to a unique couple patient / implant, and that such permanent information is associated to a file containing all updated information, by radiologist(s), about the life of this couple patient / implant and contained in a preferably protected database.

Also described herein is an implant placed or to be placed into a human body, comprising at least one passive electronic component which: is embedded in said implant, contains a memory on which is recorded some information related to said implant, so that said recorded information is wirelessly readable by a reader external to said body without intervention of any active electronic component located or to be located in said body.

Furthermore described herein is a breast implant containing a memory on which is recorded some wirelessly readable information from which said breast implant response to imaging signal is derivable. Response to imaging signal will be radio-opacity in case of X-ray imaging.

The object of the invention is also achieved with an imaging system suitable for use with an implant placed into a human body, being external to said body and comprising: a reader external to said body, adapted to read some information related to said implant and recorded in a memory embedded in said implant, an imaging device adapted to change, according to said read information, at least one of its imaging parameters, said imaging device being also adapted to image said implant with said changed imaging parameter(s).

According to a preferred embodiment of the invention, a tracking device, embedded in an implant, is associated to an imaging equipment capable of collecting information from the tracking device, in order to plan and to optimize the imaging of the implant. Information about the implant characteristics is recorded in the tracking device which is preferably a chip or a microchip. The implant is regularly controlled to check its integrity once placed into the human body, through imaging of the organ that contains the implant. The imaging equipment containing a receiver, which collects wirelessly the information recorded in the tracking device, is associated along with the implant embedding the tracking device. The imaging equipment then makes use of this information about the implant characteristics to select the best imaging parameters for imaging this implant.

Among technical and commercial advantages of this preferred embodiment of the invention, there are:
- increased quality of the image of the implant, as the imaging parameters are tailored to the implant characteristics, which can be very variable from one implant to another;
- minor modifications of the imaging system, as the (micro)chip reader can be designed as an accessory add-on device to the basic imaging system which only needs some added programming;
- improved usability of the imaging system in the case of implants, as optimal image acquisition can be fully automated, tailored to each imaged subject, and involves minimal input from the users who are the patient and the radiologist or imaging specialist.

An alternative method to solve the problem of adapting imaging parameters to the implant characteristics could be based on pre-imaging of the implant to derive its characteristics from this pre-image. Then, the imaging system would similarly optimize its imaging parameters based on the implant characteristics. However, this alternative method would present some disadvantages, among which there are:
- the need for an additional image, that could involve longer examination time, and in some cases, increased patient radiation dose;
- no guarantee of a correct derivation of proper implant characteristics based on the pre-image processing.

Preferred embodiments comprise one or more of the following features, which can be taken separately or together, either in partial combination or in full combination. These preferred embodiments can be combined with any of the preceding objects of the invention, as well as any of the following other objects of the invention.

Preferably, said reading is wireless. Wireless reading enables reading of information embedded in the implant, without any intrusion within the human body into which the implant has been placed.

Preferably, said imaging is followed by checking integrity of said implant. Any weakness or sign of weakness in the structure of the implant can be seen that way relatively early, so that intervention on the implant can be performed before the human body suffers or undergoes difficulties or damages. An integrity failure in the external surface of the implant can be seen on the image of the implant, at least by an imaging specialist, either directly or through modification of the shape of the imaged implant, or even for example by liquid serum drops position within a breast implant where they should not be in case of a breast implant.

Preferably, said reader is connected to said imaging device. Information collected by the reader can be directly transmitted to the imaging device which can make use of them early, even before performing first imaging, thereby readily producing an image with optimal quality based on the information collected on the implant.

Preferably, said implant is completely embedded under a skin of said human body. That makes a wireless reading of information embedded in the implant all the more interesting. That also makes any direct visual reading of information on the external surface of the implant impossible, what makes the readable information embedded in the implant all the more interesting.

Preferably, said implant is breast implant, that is to say breast prosthesis. Breast implant can be constituted with very different constituent materials, presenting very different responses to imaging signal, especially to X-ray radiation. Therefore, to adapt one or more of the characteristics of the imaging signal, for example wavelength and intensity, to the minimum level required for an image of good quality, it is very interesting to know beforehand the type of breast implant. Breast implant may be of at least three different types: a breast implant containing essentially silicone gel, a breast implant containing essentially saline solution, a breast implant containing essentially a combination of a silicone gel and a saline solution.

Preferably, said memory is included in a chip, preferably in a microchip, or even in a lab-on-a-chip. A chip allows for little required space to implement the memory, a microchip for even less required space, taken into account that the useful information to be stored, although very important for subsequent imaging, does require only low storage capacity of the memory. Including said memory in a lab-on-a-chip would allow for making some tests directly in situ in the human body to be able to real time check the integrity of the implant. For example, intrusion of external liquid serum within a breast implant would mean there is a crack in the external surface of the implant.

Preferably, said chip is a passive electronic component. That way, the interactions between implant and human body are limited, so that tolerance of the human body to the implant is higher. In case of failure of the passive electronic component, risk for the human body is much more limited than in case of an active electronic component which includes a source of energy, which can send signals, which can initiate some action.

Preferably, said chip is a RFID chip. This chip is small, passive and practical, so its intrusion into human body is somewhat minimal.

Preferably, said chip is fixed on an external surface of said implant. That way, internal structure does not need to be changed. More preferably, said chip is embedded under an external surface of said implant. That way, there is no supplementary intrusion in the human body, as compared to an implant void of any recorded information. Avoiding any direct contact between chip and human body ensures better tolerance of the human body to this chip.

Preferably, said recorded information includes permanent information comprising one or more initial characteristics of said implant, from which permanent information said implant response to imaging signal is derivable, among which permanent information preferably a composition of said implant and/or a size of said implant and/or a localization of said implant and/or a shape of said implant is or are derivable. Deriving response to imaging signal is very interesting since that allows for adapting, right from the beginning, the required level of imaging signal to be sent to the human body for performing adequate imaging, not less than what is needed to avoid making supplementary imaging what would lead in turn to higher imaging signal level than strictly needed, and not more than what would lead to higher imaging signal level than strictly needed. More important is information related to the composition of the implant, then not as important is information related to the size of the implant, because both have consequences on the response to imaging signal, and then less important, are information related to the location of the implant and to the shape of the implant, because both have consequences more on the position of the implant within the human body. Some composition of implant may imply several types of different imaging, whereas other composition of implant may on the contrary imply only a single type of imaging: it is very interesting to know that, for the imaging specialist or for the imaging system, before starting imaging.

Preferably, said recorded information includes time dependent information preferably comprising one or more evolving characteristics of said implant and/or one or more former control dates and/or one or more future control dates. This would help the monitoring of the implant to be performed efficiently during due course of time, one imaging specialist taking benefit on the one hand from the information initially recorded as well as on the other hand from the information recorded by him or by another imaging specialist during preceding controls of the implant. This information can also be taken into account directly by the imaging system.

Preferably, said recorded time dependent information has been recorded by a device external to said body. The information recording has been performed by the imaging specialist, and that way no active electronic component is needed within the implant. One can also contemplate that this recorded information could be read by a reader connected to the personal computer of the patient, so that reminders can be displayed to the patient in case of a coming or forgotten control to perform in case of a passive electronic component, or alerts can be displayed to the patient or even sent directly to doctor or to hospital in case there is an integrity failure in the implant that has been measured by the lab-on-a chip contained in the implant.

Preferably, said imaging is X-ray imaging and/or ultrasound imaging and/or magnetic resonance imaging. The fact of knowing beforehand about the type of implant, because of more critical radiopacity level or radiation transparency, is especially critical for X-ray imaging, important for ultrasound, and less important for magnetic resonance imaging.

Preferably, said adapted imaging parameter(s) comprise(s) nature of imaging signal and/or energy level of imaging signal, for example wavelength of imaging radiation and/or intensity of imaging radiation. In case of X-ray imaging, depending on the type of implant, some wavelengths will go through more or less easily, and the needed radiation intensity to perform a good and satisfactory imaging will be variable too.

According to another aspect of the disclosure, it is proposed a breast implant containing a memory on which is recorded some wirelessly readable information.

According to still another aspect of the disclosure, it is proposed a breast implant embedding a chip (or a microchip) wirelessly readable and/or writable. Preferably, said chip or said microchip is a passive electronic component, which means it contains no energy source. It is for example an RFID chip, which can only answer message emitted by an external device, but not take the initiative of sending itself a message toward such external device.

Further features and advantages of the invention will appear from the following description of embodiments of the invention, given as nonlimiting examples, with reference to the accompanying drawings listed hereunder.

### BRIEF DESCRIPTION OF THE DRAWINGS

Fig. 1 shows an example of performing the steps of the method of imaging an implant placed into a human body, according to a preferred embodiment of the invention.
Fig. 2 shows an example of an imaging system adapted to perform the steps of the method of imaging an implant placed into a human body, according to a preferred embodiment of the invention.

### DETAILED DESCRIPTION OF THE INVENTION

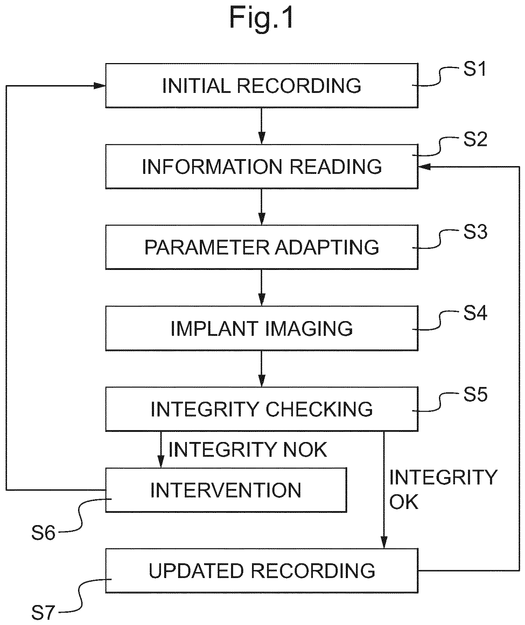

Fig. 1 shows an example of performing the steps of the method of imaging an implant placed into a human body, according to a preferred embodiment of the invention.

An example of performing the steps of the method of imaging an implant placed into a human body, according to a preferred embodiment of the invention, includes performing successively the following steps: a step of initial recording S1, a step of information reading S2, a step of parameter adapting S3, a step of implant imaging S4, a step of integrity checking S5. Depending on the results of the step S5 of integrity checking, either an intervention step S6 is performed, followed by a come back to initial recording step S1 with a new implant, or an updated recording step S7 is performed, followed by a come back to information reading step S2. So, this method is a cycling process. Steps S1 to S7 will be now described more in detail. The considered implant will be a breast implant by way of example only. The memory on which information is recorded will be included in a chip by way of example only. References will be made to the chip including the memory or the memory itself indifferently.

In initial recording step S1, when a breast implant is manufactured, a chip containing a memory is embedded in the breast implant. One or more information, preferably about breast implant composition (constituent material(s)), about breast implant size, about breast implant intended location, about breast implant shape, is initially recorded in the memory contained in the chip embedded in the breast implant. Then, the breast implant will be placed into the human body of a patient which will usually be a woman for a breast implant. The process then goes to information reading step S2.

In information reading step S2, when the patient goes to an imaging specialist, information recorded on the chip embedded in the breast implant will be read by a reader external to the patient, so that recorded information can be extracted. The process then goes to parameter adapting step S3.

In parameter adapting step S3, depending on the content of the read information, some imaging parameters will be adapted. For example, in case of X-ray imaging, for a silicone gel breast implant, rather opaque to X-ray radiation, the energy level of the radiation source of the imaging device will be increased, whereas for a saline solution breast implant, rather transparent to X-ray radiation, the energy level of the radiation source of the imaging device will be kept limited. This or those imaging parameters are changed depending on the read information which has then a direct influence on them. The process then goes to implant imaging step S4.

In implant imaging step S4, the breast implant is imaged by the imaging device tailored with the adapted or changed imaging parameters which have been determined in preceding parameter adapting step S3. The resulting image is then optimized with respect to the type of breast implant which has been imaged, since the imaging parameters of the imaging device have been adapted to this type of breast implant. The process then goes to integrity checking step S5.

In integrity checking step S5, an analysis of the resulting optimized image of the breast implant is made. Either this analysis gives a positive result, implying that the integrity of the breast implant is OK and then process goes to updated recording step S7, or this analysis gives a negative result, implying that the integrity of the breast implant is not OK and then the process goes to intervention step S6.

In intervention step S6, the defective breast implant is removed from the human body of the patient. A new, correct, maybe or preferably of different type, breast implant will be placed in the human body of the patient instead. This is done through an operation which follows the performing of the imaging method. This intervention step S6 is not included in the imaging method; it comes after performing the imaging method if the result of the imaging method is negative with respect to integrity of the breast implant. The process then comes back to initial recording step S1 which is performed for the new breast implant manufactured and placed into the human body of the patient instead of the former defective breast implant which has been removed earlier in intervention step S6.

In updated recording step S7, new information about evolution of the breast implant which integrity is not endangered, and information about performed control, such as its date and positive result, and information about next scheduled control to monitor safely integrity of the breast implant, are recorded in the chip embedded in the breast implant. Then, in the chip embedded in the breast implant, can be found both initial permanent information and time dependent updated information. The process then comes back to information reading step S2 and can continue to iterate accordingly.

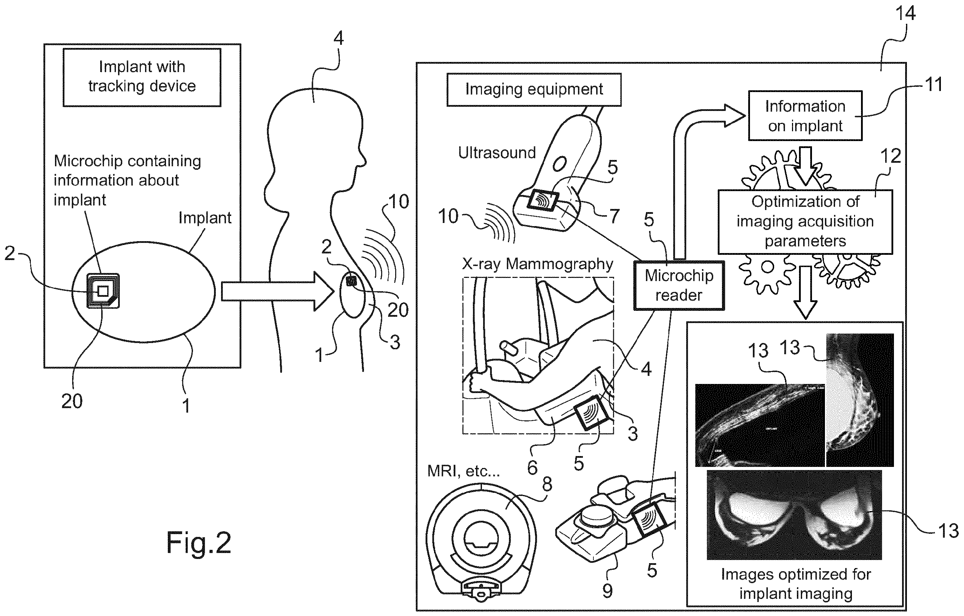

Fig. 2 shows an example of an imaging system adapted to perform the steps of the method of imaging an implant placed into a human body, according to a preferred embodiment of the invention. The considered implant will be a breast implant by way of example only. The memory on which information is recorded will be included in a chip by way of example only. References will be made to the chip including the memory or the memory itself indifferently.

A breast implant 1 encompasses a chip 2. The chip 2 is embedded in the breast implant 1. The chip 2 contains a memory 20 on which information related to breast implant 1 is recorded. From this information, the radiopacity level or the radiation transparency to imaging radiations can be derived, either by the imaging system which will than adapt accordingly or by the imaging specialist which will then tailor the imaging system accordingly. The chip 2 can be considered as a tracking device in the meaning that it enables tracking information related to breast implant, thereby allowing for monitoring the integrity of the breast implant.

The breast implant 1 is placed into the breast 3 of the human body 4 of a woman.

The imaging system 14 is completely external to the human body 4 of the patient. The imaging system 14 may comprise one or more of imaging devices such as an X-ray imaging device 6 associated with an external reader 5, an ultrasound imaging device 7 associated with an external reader 5, a Resonance Magnetic Imaging device 8 associated with a Radio Frequency device 9 including an external reader 5. The reader 5 which is external to the human body 4 of the woman, as well as external to the breast implant 1, and which is preferably an add-on device to the imaging device, 6, 7 or 8 and 9, will be called the external reader 5. This external reader 5 could be integrated in the imaging device 6, 7 or 8 and 9, too.

The external reader 5 will wirelessly communicate with the chip or microchip 2 which is preferably a passive electronic component, for example a RFID chip. The signal emission will be performed by the external reader 5. This external reader 5 will read information recorded on the memory 20 of the chip 2 and will extract information 11 related to breast implant 1. Radiopacity or response to imaging signal is derivable from this relevant information 11 related to breast implant 1. According to this derived radiopacity or response to imaging signal, the imaging parameters 12, preferably imaging acquisition parameters 12, will be changed and optimized with respect to the specific breast implant 1.

Then, once the imaging device 6, 7 or 8 and 9, has changed its imaging parameters 12, the images 13 of the breast implant 1 are made or acquired; these images 13 of the breast implant 1 are optimized with respect to this specific imaged breast implant 1, thanks to beforehand optimization of the imaging parameters 12.

The invention has been described with reference to preferred embodiments. However, many variations are possible within the scope of the invention.

## Claims

1. A method of imaging an implant (1) placed into a human body (4), comprising:
- reading (S2), with a reader (5) external to said body (4), information (11) related to said implant (1) and recorded in a memory (20) embedded in said implant (1),
- adapting (S3), according to said read information (11), at least one imaging parameter (12) of an imaging device (6, 7, 8, 9) external to said body (4),
- imaging (S4), by said imaging device (6, 7, 8, 9) with said adapted imaging parameter(s) (12), said implant (1).

2. A method of imaging an implant (1) placed into a human body (4), according to claim 1, wherein said reading (S2) is wireless.

3. A method of imaging an implant (1) placed into a human body (4), according to any of preceding claims, wherein said imaging (S4) is followed by checking integrity (S5) of said implant (1).

4. A method of imaging an implant (1) placed into a human body (4), according to any of preceding claims, wherein said implant (1) is completely embedded under a skin of said human body (4).

5. A method of imaging an implant (1) placed into a human body (4), according to any of preceding claims, wherein said implant (1) is a breast implant.

6. A method of imaging an implant (1) placed into a human body (4), according to any of preceding claims, wherein said memory (20) is included in a chip (2) which is a passive electronic component, which is preferably a RFID chip.

7. A method of imaging an implant (1) placed into a human body (4), according to any of claims 1 to 6, wherein said memory (20) is included in a chip (2) which is fixed on an external surface of said implant (1).

8. A method of imaging an implant (1) placed into a human body (4), according to any of claims 1 to 6, wherein said memory (20) is included in a chip (2) which is embedded under an external surface of said implant (1).

9. A method of imaging an implant (1) placed into a human body (4), according to any of preceding claims, wherein said recorded information (11) includes permanent information comprising one or more initial characteristics of said implant (1), from which permanent information said implant response to imaging signal is derivable, among which permanent information preferably a composition of said implant (1) and/or a size of said implant (1) and/or a localization of said implant (1) and/or a shape of said implant (1) is or are derivable.

10. A method of imaging an implant (1) placed into a human body (4), according to any of preceding claims, wherein said recorded information (11) includes time dependent information recorded in said memory (20) either initially or by an imaging specialist by means of a device (5) external to said body (4), said time dependent information preferably comprising one or more evolving characteristics of said implant (1) and/or one or more former control dates and/or one or more future control dates.

11. A method of imaging an implant (1) placed into a human body (4), according to claim 10, wherein said recorded time dependent information has been recorded by a device (5) external to said body (4).

12. A method of imaging an implant (1) placed into a human body (4), according to any of preceding claims, wherein said imaging (S4) is X-ray imaging and/or ultrasound imaging and/or magnetic resonance imaging.

13. A method of imaging an implant (1) placed into a human body (4), according to any of preceding claims, wherein said adapted imaging parameter(s) (12) comprise(s) nature of imaging signal and/or energy level of imaging signal.

14. A method of imaging an implant (1) placed into a human body (4), according to any of preceding claims, wherein said memory (20) is writable by a device (5) external to said body (4).

15. An imaging system (14) suitable for use with an implant (1) placed into a human body (4), being external to said body (4) and comprising:
- a reader (5) external to said body (4), adapted to read some information (11) related to said implant (1) and recorded in a memory (20) embedded in said implant (1),
- an imaging device (6, 7, 8, 9) adapted to change, according to said read information (11), at least one of its imaging parameters (12), said imaging device (6, 7, 8, 9) being also adapted to image said implant (1) with said changed imaging parameter(s) (12).

## Patentansprüche

1. Das Verfahren zum Abbilden eines Implantats (1), das in einem menschlichen Körper (4) angeordnet ist, umfassend:
- Lesen (S2), mit einem Lesegerät (5) außerhalb des Körpers (4), von Information (11), die das Implantat (1) betrifft und die in einem Speicher (20), welcher im Implantat (1) eingebettet ist, aufgezeichnet ist,
- Anpassen (S3), gemäß der gelesenen Information (11), von zumindest einem Abbildungsparameter (12) eines Abbildungsgeräts (6, 7, 8, 9) außerhalb des Körpers (4),
- Abbilden (S4), durch das Abbildungsgerät (6, 7, 8, 9) mit dem (den) angepassten Parameter(n) (12), des Implantats (1).

2. Das Verfahren zum Abbilden eines Implantats (1), das in einem menschlichen Körper (4) angeordnet ist, nach Anspruch 1, wobei das Lesen (S2) drahtlos erfolgt.

3. Das Verfahren zum Abbilden eines Implantats (1), das in einem menschlichen Körper (4) angeordnet ist, nach einem der vorhergehenden Ansprüche, wobei dem Abbilden (4) das Überprüfen der Unversehrtheit (S5) des Implantats (1) folgt.

4. Das Verfahren zum Abbilden eines Implantats (1), das in einem menschlichen Körper (4) angeordnet ist, nach einem der vorhergehenden Ansprüche, wobei das Implantat (1) vollständig unter der Haut des menschlichen Körpers (4) eingebettet ist.

5. Das Verfahren zum Abbilden eines Implantats (1), das in einem menschlichen Körper (4) angeordnet ist, nach einem der vorhergehenden Ansprüche, wobei das Implantat (1) ein Brustimplantat ist.

6. Das Verfahren zum Abbilden eines Implantats (1), das in einem menschlichen Körper (4) angeordnet ist, nach einem der vorhergehenden Ansprüche, wobei der Speicher (20) in einem Chip (2) enthalten ist, der ein passives elektronisches Bauteil ist, welches vorzugsweise ein RFID-Chip ist.

7. Das Verfahren zum Abbilden eines Implantats (1), das in einem menschlichen Körper (4) angeordnet ist, nach einem der Ansprüche 1 bis 6, wobei der Speicher (20) in einem Chip (2) enthalten ist, der an einer Außenfläche des Implantats (1) befestigt ist.

8. Das Verfahren zum Abbilden eines Implantats (1), das in einem menschlichen Körper (4) angeordnet ist, nach einem der Ansprüche 1 bis 6, wobei der Speicher (20) in einem Chip (2) enthalten ist, der unter einer Außenfläche des Implantats (1) eingebettet ist.

9. Das Verfahren zum Abbilden eines Implantats (1), das in einem menschlichen Körper (4) angeordnet ist, nach einem der vorhergehenden Ansprüche, wobei die aufgezeichnete Information (11) permanente Information enthält, die eine oder mehr anfängliche Kennzeichen des Implantats (1) umfasst, wobei aus der permanenten Information die Implantatreaktion auf ein Abbildungssignal ableitbar ist, wobei aus der permanenten Information vorzugsweise eine Zusammensetzung des Implantats (1) und/oder eine Größe des Implantats (1) und/oder eine Lage des Implantats (1) und/oder eine Form des Implantats (1) ableitbar ist

10. Das Verfahren zum Abbilden eines Implantats (1), das in einem menschlichen Körper (4) angeordnet ist, nach einem der vorhergehenden Ansprüche, wobei die aufgezeichnete Information (11) zeitabhängige Information enthält, die im Speicher (20) entweder anfangs oder durch einen Abbildungsspezialisten mittels eines Geräts (5) außerhalb des Körpers (4) aufgezeichnet wird, wobei die zeitabhängige Information vorzugsweise ein oder mehr Entwicklungskennzeichen des Implantats (1) und/oder ein oder mehr frühere Steuerdaten und/oder ein oder mehr künftige Steuerdaten umfasst.

11. Das Verfahren zum Abbilden eines Implantats (1), das in einem menschlichen Körper (4) angeordnet ist, nach Anspruch 10, wobei die aufgezeichnete zeitabhängige Information durch ein Gerät (5) außerhalb des Körpers (4) aufgezeichnet wurde.

12. Das Verfahren zum Abbilden eines Implantats (1), das in einem menschlichen Körper (4) angeordnet ist, nach einem der vorhergehenden Ansprüche, wobei das Abbilden (S4) Röntgenstrahlenabbilden und/oder Ultraschallabbilden und/oder Magnetresonanzabbilden ist.

13. Das Verfahren zum Abbilden eines Implantats (1), das in einem menschlichen Körper (4) angeordnet ist, nach einem der vorhergehenden Ansprüche, wobei der (die) angepassten Abbildungsparameter (12) Abbildungssignalbeschaffenheit und/oder Abbildungssignalenergiepegel umfasst (umfassen).

14. Das Verfahren zum Abbilden eines Implantats (1), das in einem menschlichen Körper (4) angeordnet ist, nach einem der vorhergehenden Ansprüche, wobei der Speicher (20) durch ein Gerät (5) außerhalb des Körpers (4) beschreibbar ist.

15. Abbildungssystem (14) das zum Gebrauch mit einem Implantat (1), welches in einem menschlichen Körper (4) angeordnet ist, geeignet ist, außerhalb des Körpers (4) liegt und umfasst:
- ein Lesegerät (5) außerhalb des Körpers (4), das zum Lesen von Information (11), die das Implantat (1) betrifft und in einem Speicher (20), welcher im Implantat (1) eingebettet ist, aufgezeichnet ist, geeignet ist,
- ein Abbildungsgerät (6, 7, 8, 9), das zum Ändern, gemäß der gelesenen Information (11), von zumindest einem seiner Abbildungsparameter (12) geeignet ist, wobei das Abbildungsgerät (6, 7, 8, 9) außerdem zum Abbilden des Implantats (1) mit dem (den) geänderten Abbildungsparameter(n) (12) geeignet ist.

## Revendications

1. Procédé d'imagerie d'un implant (1) placé dans un corps humain (4), comprenant les étapes consistant à :
- lire (S2), à l'aide d'un lecteur (5) externe audit corps (4), des informations (11) relatives audit implant (1) et enregistrées dans une mémoire (20) intégrée dans ledit implant (1),
- adapter (S3), selon lesdites informations lues (11), au moins un paramètre d'imagerie (12) d'un dispositif d'imagerie (6, 7, 8, 9) externe audit corps (4),
- former une image (S4), par ledit dispositif d'imagerie (6, 7, 8, 9) avec ledit/lesdits paramètre(s) d'imagerie adapté(s) (12), dudit implant (1).

2. Procédé d'imagerie d'un implant (1) placé dans un corps humain (4), selon la revendication 1, dans lequel ladite lecture (S2) se fait sans fil.

3. Procédé d'imagerie d'un implant (1) placé dans un corps humain (4), selon l'une quelconque des revendications précédentes, dans lequel ladite imagerie (S4) est suivie d'une vérification de l'intégrité (S5) dudit implant (1).

4. Procédé d'imagerie d'un implant (1) placé dans un corps humain (4), selon l'une quelconque des revendications précédentes, dans lequel ledit implant (1) est complètement intégré sous une peau dudit corps humain (4).

5. Procédé d'imagerie d'un implant (1) placé dans un corps humain (4), selon l'une quelconque des revendications précédentes, dans lequel ledit implant (1) est un implant mammaire.

6. Procédé d'imagerie d'un implant (1) placé dans un corps humain (4), selon l'une quelconque des revendications précédentes, dans lequel ladite mémoire (20) est incluse dans une puce (2) qui est un composant électronique passif, qui est de préférence une puce d'identification par radiofréquence.

7. Procédé d'imagerie d'un implant (1) placé dans un corps humain (4), selon l'une des revendications 1 à 6, dans lequel ladite mémoire (20) est incluse dans une puce (2) qui est fixée sur une surface externe dudit implant (1).

8. Procédé d'imagerie d'un implant (1) placé dans un corps humain (4), selon l'une des revendications 1 à 6, dans lequel ladite mémoire (20) est incluse dans une puce (2) qui est intégrée sous une surface externe dudit implant (1).

9. Procédé d'imagerie d'un implant (1) placé dans un corps humain (4), selon l'une quelconque des revendications précédentes, dans lequel lesdites informations enregistrées (11) comportent des informations permanentes comprenant une ou plusieurs caractéristiques initiales dudit implant (1), informations permanentes à partir desquelles est dérivable une réponse dudit implant à un signal d'imagerie, informations permanentes parmi lesquelles est ou sont dérivable(s), de préférence, une composition dudit implant (1) et/ou une taille dudit implant (1) et/ou une localisation dudit implant (1) et/ou une forme dudit implant (1).

10. Procédé d'imagerie d'un implant (1) placé dans un corps humain (4), selon l'une quelconque des revendications précédentes, dans lequel lesdites informations enregistrées (11) comportent des informations dépendantes du temps enregistrées dans ladite mémoire (20) soit initialement soit par un spécialiste en imagerie au moyen d'un dispositif (5) externe audit corps (4), lesdites informations dépendantes du temps comprenant de préférence une ou plusieurs caractéristiques évolutives dudit implant (1) et/ou une ou plusieurs anciennes dates de commande et/ou une ou plusieurs futures dates de commande.

11. Procédé d'imagerie d'un implant (1) placé dans un corps humain (4), selon la revendication 10, dans lequel lesdites informations dépendantes du temps enregistrées ont été enregistrées par un dispositif (5) externe audit corps (4).

12. Procédé d'imagerie d'un implant (1) placé dans un corps humain (4), selon l'une quelconque des revendications précédentes, dans lequel ladite imagerie (S4) est une imagerie aux rayons X et/ou une imagerie aux ultrasons et/ou une imagerie par résonance magnétique.

13. Procédé d'imagerie d'un implant (1) placé dans un corps humain (4), selon l'une quelconque des revendications précédentes, dans lequel ledit/lesdits paramètre(s) d'imagerie adapté(s) (12) comprend/comprennent une nature de signal d'imagerie et/ou un niveau d'énergie de signal d'imagerie.

14. Procédé d'imagerie d'un implant (1) placé dans un corps humain (4), selon l'une quelconque des revendications, dans lequel ladite mémoire (20) est inscriptible par un dispositif (5) externe audit corps (4).

15. Système d'imagerie (14) approprié pour une utilisation avec un implant (1) placé dans un corps humain (4), étant externe audit corps (4) et comprenant :
- un lecteur (5) externe audit corps (4), adapté pour lire certaines informations (11) relatives audit implant (1) et enregistrées dans une mémoire (20) intégrée dans ledit implant (1),
- un dispositif d'imagerie (6, 7, 8, 9) adapté pour modifier, selon lesdites informations lues (11), au moins au moins l'un de ses paramètres d'imagerie (12), ledit dispositif d'imagerie (6, 7, 8, 9) étant également adapté pour former une image dudit implant (1) avec ledit/lesdits paramètre(s) d'imagerie modifié(s) (12).
